# EUROPEAN PATENT SPECIFICATION

(11) **EP 4 387 397 B1**
(45) Date of publication and mention of the grant of the patent: **19.11.2025**
(21) Application number: 23216014.3
(22) Date of filing: 12.12.2023
(51) Int. Cl.: H05B 47/115

(54) **SYSTEMS AND METHODS FOR CONTROLLING A MEDICAL LIGHT VIA A HANDLE**
SYSTEME UND VERFAHREN ZUR STEUERUNG EINER MEDIZINISCHEN LEUCHTE ÜBER EINEN GRIFF
SYSTÈMES ET PROCÉDÉS DE COMMANDE D'UNE LUMIÈRE MÉDICALE PAR L'INTERMÉDIAIRE D'UNE POIGNÉE

(30) Priority: 12.12.2022 US 202263387085 P
(43) Date of publication of application: 19.06.2024
(60) Divisional application: 25206647.7
(73) Proprietor: Stryker Corporation, Portage, MI 49002-9711 (US)
(72) Inventor: MUSKO, John Edward, Kalamazoo, 49002 (US); SIERRA ROMERO, Alberto, Kalamazoo, 49002 (US)
(74) Representative: V.O.

(56) References cited:
- US-A1- 2012 075 832
- US-B2- 9 920 922

## Description

### FIELD

The present disclosure relates generally to medical lighting, and more specifically to controlling characteristics of illumination light provided by a medical light.

### BACKGROUND

US 2012/0075832 discloses a surgical lamp with a contactless adjustment of the focus or light intensity of the lamp via an operating device in a handle of the lamp. Repositioning of the lamp is recognized by an acceleration sensor in the lamp body. During repositioning, the operating device is controlled so that it does not react to the signals produced by contactless sensors during repositioning, so that no signals are passed to the focusing device and/or dimming device.

Medical lights can be used in operating rooms to illuminate a desired area of a patient undergoing a surgical procedure. Medical lights are often mounted on suspension arms that enable users to adjust the position and orientation of the medical lights. Medical lights often have a centrally located handle that a user can grasp to reposition the medical light. In addition to providing a means for repositioning the medical light, the centrally located handle can also include one or more user interfaces for adjusting attributes of the light provided by the medical light, such as intensity, spot size, color temperature, and spectral distribution. One advantage of adjusting such features using the centrally located handle is that the handle can include a sterile cover such that the surgeon can adjust attributes of the light without breaking sterility. However, because the handle can be used both for adjusting attributes of the illumination light and for repositioning the medical light, the surgeon may inadvertently adjust attributes of the light while repositioning the medical light.

### SUMMARY

A method for controlling a medical light according to the disclosure comprises:
determining that the medical light is being repositioned based on a signal from at least one movement sensor; and
in response to determining that the medical light is being repositioned:
   disabling adjustment of at least one function of the medical light by at least one user input interface of a handle assembly of the medical light, comprising while the medical light is being repositioned, receiving a user input via the at least one user input interface to adjust the at least one function of the medical light; and
   ignoring the user input to adjust the at least one function of the medical light; or
   enabling adjustment of a setting of at least one function while the medical light is being repositioned comprising receiving a user input via the at least one user input interface to adjust the at least one function of the medical light, and in response to determining that the medical light is no longer being repositioned return the at least one function of the light back to its setting prior to the medical light being repositioned.

Systems, devices, and methods described herein prevent or reverse inadvertent adjustment of a function of a medical light while the medical light is being repositioned by a user using the handle assembly. The medical light may include at least one movement sensor configured to detect movement of the medical light and a controller that determines the medical light is being repositioned based on detected movement. In response to determining that the medical light is being repositioned, the controller may disable adjustment of at least one function of the medical light by at least one user input interface of the handle assembly. Alternatively, the controller may enable adjustment of a setting of at least one function while the medical light is being repositioned but return the at least one function of the light back to its setting prior to the medical light being repositioned.

Optionally, the at least one function of the medical light comprises providing illumination light according to at least one setting, and disabling adjustment of the at least one function comprises disabling adjustment of the at least one setting.

The at least one setting may comprise an intensity of the illumination light provided by the medical light, a spot size of the illumination light, a color temperature of the illumination light, or a spectral distribution of the illumination light.

Optionally, the signal from the at least one movement sensor is associated with a change in angle and/or translation of the medical light.

Determining that the medical light is being repositioned may comprise determining that the change in angle and/or translation of the medical light exceeds one or more predetermined thresholds.

The at least one movement sensor may comprise an accelerometer, a gyroscope, or a position encoder.

Optionally, the user input comprises at least one of: a twist of a handle of the handle assembly, a press of a button of the handle assembly, a touch of a capacitive sensor of the handle assembly, a rotation of a dial, a slide of a slider knob, and a rotation of a ring of the handle assembly.

The at least one function may comprise a characteristic of a camera of the medical light.

Optionally, the camera is controlled by an external camera controller, and disabling adjustment of the at least one function comprises disabling communication of commands to the external camera controller.

According to an aspect, a medical light comprises : a handle assembly configured for grasping by a user for moving the medical light, the handle assembly comprising at least one user input interface configured to receive inputs from the user for adjusting at least one function of the medical light, at least one movement sensor configured to detect movement of the medical light, and a controller comprising one or more processors, memory, and one or more programs stored in the memory for execution by the one or more processors, wherein the one or more programs include instructions that, when executed by the one or more processors cause the controller to: determine that the medical light is being repositioned based on a signal from the at least one movement sensor, and in response to determining that the medical light is being repositioned, disable adjustment of the at least one function of the medical light by the at least one user input interface of the handle assembly, or enable adjustment of a setting of the at least one function while the medical light is being repositioned and in response to determining that the medical light is no longer being repositioned return the at least one function of the light back to its setting prior to the medical light being repositioned.

The controller may be located in the handle assembly.

The controller may be located in a housing of the medical light.

The medical light may comprise an arm that comprises the at least one movement sensor.

Optionally, the at least one user input interface comprises one of a twistable handle, at least one button, at least one touch sensor, at least one rotatable dial, at least one slider knob, and at least one rotatable ring.

The at least one function of the medical light may comprise providing illumination light according to at least one setting, and disabling adjustment of the at least one function comprises disabling adjustment of the at least one setting.

The at least one setting may comprise an intensity of the illumination light provided by the medical light, a spot size of the illumination light, a color temperature of the illumination light, or a spectral distribution of the illumination light.

Optionally, the signal from the at least one movement sensor is associated with a change in angle and/or position of the medical light.

According to an aspect, a method for controlling at least one characteristic of illumination light provided by a medical light comprises: emitting the illumination light by the medical light according to a first setting of the at least one characteristic of the illumination light, determining that the medical light is being repositioned based on a signal from at least one movement sensor, while the medical light is being repositioned, adjusting the at least one characteristic of the illumination light to a second setting in response to a user input to at least one user input interface of a handle assembly of the medical light, determining that the medical light is no longer being repositioned based on the signal from the at least one movement sensor, and in response to determining that the medical light is no longer being repositioned, resetting the at least one characteristic of the illumination light from the second setting to the first setting.

The method may comprise saving the first setting of the at least one characteristic of illumination light in a memory prior to determining that the medical light is being repositioned.

The method may comprise saving the first setting of the at least one characteristic of illumination light in a memory in response to determining that the medical light is being repositioned.

Optionally, the first setting of the at least one characteristic of the illumination light corresponds to a global setting for a plurality of medical lights.

The at least one characteristic of the illumination light may comprise an intensity of the illumination light, a spot size of the illumination light, a color temperature of the illumination light, or a spectral distribution of the illumination light.

According to an aspect, a medical light comprises: a handle assembly configured for grasping by a user for moving the medical light, the handle assembly comprising at least one user input interface configured to receive inputs from the user for adjusting at least one characteristic of illumination light provided by the medical light, at least one movement sensor configured to detect movement of the medical light, and a controller comprising one or more processors, memory, and one or more programs stored in the memory for execution by the one or more processors, wherein the one or more programs include instructions that, when executed by the one or more processors cause the controller to: emit the illumination light by the medical light according to a first setting of the at least one characteristic of the illumination light, determine that the medical light is being repositioned based on a signal from the at least one movement sensor, while the medical light is being repositioned, adjust the at least one characteristic of the illumination light to a second setting in response to a user input to the at least one user input interface, determine that the medical light is no longer being repositioned based on the signal from at least one movement sensor, and in response to determining that the medical light is no longer being repositioned, reset the at least one characteristic of the illumination light from the second setting to the first setting.

Optionally, the one or more programs include instructions that, when executed by the one or more processors cause the controller to save the first setting of the at least one characteristic of illumination light in a memory prior to determining that the medical light is being repositioned.

The one or more programs may include instructions that, when executed by the one or more processors cause the controller to save the first setting of the at least one characteristic of illumination light in a memory in response to determining that the medical light is being repositioned.

The first setting of at least one characteristic of the illumination light may correspond to a global setting for a plurality of medical lights.

The at least one characteristic of the illumination light may comprise an intensity of the illumination light, a spot size of the illumination light, a color temperature of the illumination light, or a spectral distribution of the illumination light.

According to an aspect, a non-transitory computer readable storage medium stores one or more programs for controlling at least one characteristic of illumination light provided by a medical light, the one or more programs comprising instructions which, when executed by a controller of a medical light, cause the controller to perform any of the preceding methods.

According to an aspect, a computer program product comprises one or more software code portions for controlling at least one characteristic of illumination light provided by a medical light, the one or more software code portions comprising instructions which, when executed by a controller of a medical light, cause the controller to perform any of the preceding methods.

It will be appreciated that any of the variations, aspects, features, and options described in view of the systems apply equally to the methods and vice versa. It will also be clear that any one or more of the above variations, aspects, features, and options can be combined.

### BRIEF DESCRIPTION OF THE FIGURES

The invention will now be described, by way of example only, with reference to the accompanying drawings, in which:
FIG. 1A shows an exemplary operating room that includes a medical light in a first position; according to an aspect of the disclosure;
FIG. 1B shows the exemplary operating room of FIG. 1A that includes the medical light assembly in a second position after being repositioned, according to an aspect of the disclosure;
FIG. 2 shows a block diagram of an exemplary medical light, according to an aspect of the disclosure;
FIG. 3 shows a functional block diagram of a system for controlling a medical light according to an aspect of the disclosure;
FIG. 4 shows a block diagram of an exemplary method for controlling at least one function of a medical light, according to an aspect of the disclosure;
FIG. 5 shows a block diagram of an exemplary method for controlling at least one characteristic of light provided by a medical light, according to an aspect of the disclosure; and
FIG. 6 shows an exemplary computing device, according to an aspect of the disclosure.

### DETAILED DESCRIPTION

In the following description of the various examples, reference is made to the accompanying drawings, in which are shown, by way of illustration, specific examples that can be practiced. The description is presented to enable one of ordinary skill in the art to make and use the invention and is provided in the context of a patent application and its requirements. Various modifications to the described examples will be readily apparent to those persons skilled in the art and the generic principles herein may be applied to other examples. Thus, the present invention is not intended to be limited to the examples shown but is to be accorded the widest scope consistent with the principles and features described herein.

Systems, devices, and methods described herein include a medical light configured to prevent or reverse inadvertent adjustment of a function of a medical light that may occur when a user grasps and manipulates a handle assembly of the medical light to reposition the medical light. The medical light includes a plurality of light sources (e.g., LEDs), and a function of the medical light is to provide illumination light according to one or more adjustable characteristics, such as intensity, spot size, color temperature, spectral distribution, and/or an on/off state of the light sources. The handle assembly provides one or more user interfaces for the user to adjust the one or more adjustable characteristics of the light. For example, the handle assembly can include a rotatable grip, one or more buttons, one or more capacitive sensors, one or more rotatable rings, one or more rotatable dials, one or more sliders, or any combination of such features that the user can use to adjust one or more characteristics of the illumination light provided by the light sources. The medical light includes a controller that can adjust the characteristics of the light sources based on user inputs to the handle assembly.

When a user grasps the handle assembly to reposition the medical light, the user may inadvertently actuate one or more of the user interfaces of the handle assembly. For example, the user may inadvertently twist the rotatable grip of the handle assembly when grasping the handle assembly to reposition the medical light. To prevent such an inadvertent actuation of one or more user interfaces of the handle assembly from adjusting one or more functions of the medical light, the controller is configured to determine that the medical light is being repositioned and to manage adjustment of the one or more functions by the one or more user interfaces of the handle assembly such that the one or more functions are the same before and after the repositioning of the medical light.

To detect repositioning of the medical light, the medical light includes one or more movement sensors (such as one or more accelerometers, gyroscopes, position encoders, etc.) configured to detect movement of the medical light. Signals from the movement sensors of the medical light can be used to determine whether the medical light is being repositioned. The signals may be analyzed to determine one or more characteristics of the movement of the medical light. The one or more characteristics of the movement of the medical light can be, for example, a change in angular orientation of the medical light or a translation of the medical light. The one or more characteristics can be compared to one or more predetermined thresholds to determine that the medical light is being repositioned by a user. A change in angular orientation of the medical light may be compared to a change in angle (angular delta) threshold, or a translation of the medical light may be compared to a translational change in position (translational delta) threshold. For example, the controller of the medical light may determine the angular rotation of the medical light by analyzing a signal from a gyroscope and compare the angular rotation of the medical light to a predetermined angular delta threshold to determine whether the angular rotation indicates the medical light is being repositioned by a user.

A controller of the medical light can be configured to determine whether the medical light is being repositioned by the user based on signals received from the movement sensor(s) and manage inadvertent adjustment of functions of the medical light while the medical light is being repositioned so that the functions of the light are the same after the medical light was repositioned as they were before the medical light was repositioned. The controller may disable user inputs via the one or more user input interfaces of the handle assembly while the medical light is being repositioned. Alternatively, the controller may reverse any potentially inadvertent adjustments made while the medical light was being repositioned by resetting one or more settings of functions of the light to the settings that the medical light had prior to being repositioned.

In the following description, it is to be understood that the singular forms "a," "an," and "the" used in the following description are intended to include the plural forms as well, unless the context clearly indicates otherwise. It is also to be understood that the term "and/or" as used herein refers to and encompasses any and all possible combinations of one or more of the associated listed items. It is further to be understood that the terms "includes, "including," "comprises," and/or "comprising," when used herein, specify the presence of stated features, integers, steps, operations, elements, components, and/or units but do not preclude the presence or addition of one or more other features, integers, steps, operations, elements, components, units, and/or groups thereof.

Certain aspects of the present invention include process steps and instructions described herein in the form of a method. It should be noted that the process steps and instructions of the present invention could be embodied in software, firmware, or hardware, and, when embodied in software, they could be downloaded to reside on, and be operated from, different platforms used by a variety of operating systems. Unless specifically stated otherwise as apparent from the following discussion, it is appreciated that, throughout the description, discussions utilizing terms such as "processing," "computing," "calculating," "determining," "displaying," or the like refer to the action and processes of a computer system, or similar electronic computing device, that manipulates and transforms data represented as physical (electronic) quantities within the computer system memories or registers or other such information storage, transmission, or display devices.

The present disclosure can relate to a networked device for performing the operations herein. This device may be specially constructed for the required purposes, or it may comprise a general-purpose computer selectively activated or reconfigured by a computer program stored in the computer. Such a computer program may be stored in a non-transitory, computer readable storage medium, such as, but not limited to, any type of disk, including floppy disks, USB flash drives, external hard drives, optical disks, CD-ROMs, magnetic-optical disks, read-only memories (ROMs), random access memories (RAMs), EPROMs, EEPROMs, magnetic or optical cards, application specific integrated circuits (ASICs), or any type of media suitable for storing electronic instructions, and each coupled to a computer system bus. Furthermore, the computers referred to in the specification may include a single processor or may be architectures employing multiple processor designs for increased computing capability.

The methods, devices, and systems described herein are not inherently related to any particular computer or other apparatus. Various general-purpose systems may also be used with programs in accordance with the teachings herein, or it may prove convenient to construct a more specialized apparatus to perform the required method steps. The required structure for a variety of these systems will appear from the description below. In addition, the present invention is not described with reference to any particular programming language. It will be appreciated that a variety of programming languages may be used to implement the teachings of the present invention as described herein.

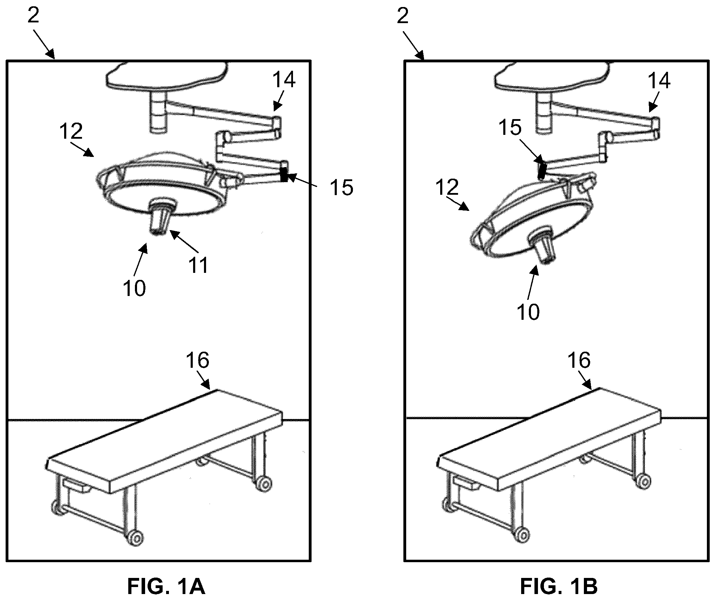

FIGS. 1A and 1B show an exemplary operating room 2 that includes a medical light 12 that is in a first position in FIG. 1A and is in a second position in FIG. 1B, such as after having been repositioned by a user. The medical light 12 provides illumination light to illuminate a patient positioned on a table 16. The medical light 12 can provide illumination light during a surgical or non-surgical procedure. When in use, it may be desirable to reposition the medical light 12 to better illuminate a particular region of the patient. The medical light 12 includes a handle assembly 10 that can be grasped by a user to move the medical light 12 as desired. For instance, to move the medical light 12 from the position shown in FIG. 1A to the position shown in FIG. 1B, a user may grasp the handle assembly 10 and physically move the medical light 12 in order to reach the position shown in FIG. 1B. The medical light 12 may include a suspension arm 14 that is connected to a support (e.g., a ceiling) to support the medical light 12 and that is configured to facilitate repositioning of the medical light 12 as desired.

The medical light 12 may be configured so that the handle assembly 10 can be used to adjust one or more functions of the medical light. For example, the handle assembly 10 may be used to adjust one or more characteristics of the illumination light provided by the medical light, such as one or more of intensity, spot size, color temperature, spectral distribution (e.g., red light, yellow light, white light, etc.), and/or an on/off state of illumination light provided by the medical light. The medical light 12 may include an in-light camera and adjustment of the one or more functions of the medical light by the handle assembly 10 can include adjusting focus, zoom, white balance, and/or an on/off state of a camera of the medical light. The handle assembly 10 can include one or more user input interfaces for receiving user inputs to adjust the one or more functions of the medical light. For example, the handle assembly 10 can include a rotatable grip 11 that a user can rotate to adjust one or more characteristics of light. For example, the user may increase the spot size of the illumination light provided by the medical light by rotating the rotatable grip 11 one way and decrease the spot size by rotating the rotatable grip 11 the opposite way.

When grasping the handle assembly 10 to reposition the medical light 12, the user may inadvertently actuate one or more user interfaces of the handle assembly 10. For example, the user may unintentionally rotate the rotatable grip 11 while grasping the handle assembly 10 to reposition the medical light 12. To prevent such an inadvertent actuation of one or more user interfaces of the handle assembly, the medical light 12 can be configured to determine when the medical light 12 is being repositioned based on signals received from one or more movement sensors that detect movement of the medical light 12, and to manage adjustment of the one or more functions of the medical light by the one or more user interfaces of the handle assembly 10 such that the one or more functions of the medical light are the same before and after the repositioning of the medical light 12.

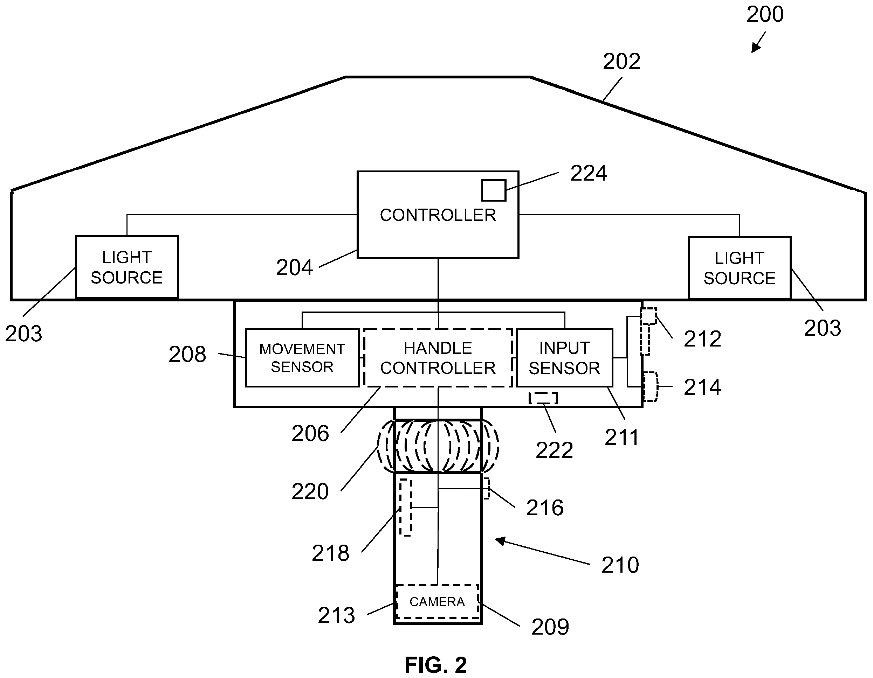

FIG. 2 shows a block diagram of an exemplary medical light 200 (e.g., medical light 12 of FIG. 1A) that is configured to manage inadvertent actuations of one or more user inputs of a handle assembly 210 during repositioning of the medical light 200. The medical light 200 can be used, for example, as medical light 12 of FIG. 1. The medical light 200 includes a plurality of light sources 203 that emit light according to one or more adjustable characteristics such as intensity, spot size, color temperature, spectral distribution, and an on/off state. The handle assembly 210 can include one or more user interfaces that a user can interact with to adjust the one or more characteristics of light provided by the medical light 200. The one or more user interfaces can include, for example, a rotatable grip 209, one or more buttons 216, one or more capacitive sensors 218, one or more rotatable rings 220, one or more rotatable dials 214, and/or one or more sliders 212.

The handle assembly 210 can include one or more input sensors 211 that detect inputs to user input interfaces of the handle assembly 210. For instance, the one or more input sensors 211 can be configured to detect a twist of the rotatable grip 209 of the handle assembly 210. Similarly, one or more input sensors 211 can be configured to detect a press of the button 216, a touch to the capacitive sensor 218, rotation of the dial 214, sliding of the slider 212, and/or a rotation of the ring 220. The location of the one or more input sensors 211 in FIG. 2 is merely exemplary in nature and it will be understood by a person of ordinary skill in the art that the one or more input sensors 211 can be located in any suitable location of the handle assembly 210 depending on the type and location of user interfaces. The one or more input sensors 211 can communicate signals corresponding to user inputs to one or more user input interfaces to the controller 204, which can control the light sources 203 to adjust one or more characteristics of the light based on inputs to one or more of the user input interfaces. The controller 204 may save settings related to the adjustable characteristics of the light sources 203 in a memory 224.

The medical light 200 may include a handle controller 206 that receives inputs from the input sensor 211 and sends commands to the controller 204 that instruct the controller 204 regarding which light characteristic should be adjusted for a given user input interface actuation. For example, the handle controller 206 may send an "increase intensity" command to the controller 204 in response to the one or more input sensors 211 detecting a twist of the hand grip 209 of the handle assembly 210, and the controller 204 may respond to this command by increasing the intensity of the illumination light provided by the medical light 200. The handle controller 206 may communicate which user input was received (e.g., a press of button 216), or alternatively, which adjustment should be made (e.g., turn off the light sources 203) to the controller 204. Configuration data that specifies mappings of certain handle actuations to specific light adjustment commands may be stored in a memory (not shown in figure) of the handle controller 206.

The handle assembly 210 may include a camera 213. The camera 213 can be operated according to one or more adjustable characteristics that may be controlled by controller 204 or handle controller 206, or via an external controller. The adjustable characteristics of the camera 213 can include, for example, controlling focus of the camera, turning the camera on or off, controlling the zoom of the camera, and controlling the white balance of the camera. One or more user input interfaces of the medical light 200 can be used to control the camera 213.

One or more movement sensors 208 can be integrated into the medical light 200 and/or into the suspension arm of the medical light 200 (see, for example, movement sensor 15 integrated into suspension arm 14 of FIG. 1). One or more movement sensors 208 integrated into the medical light 200 can be located within a housing 202 of the medical light 200, within the handle assembly 210, and/or in any other suitable location. The movement sensors 208 are configured to detect movement of the medical light 200. The movement sensors 208 can include one or more accelerometers, gyroscopes, position encoders, etc., that can detect translational movement, rotational movement, or both. One or more movement sensors 208 integrated into a suspension arm of the medical light 200 can include one or more encoders integrated into joints of the suspension arm that detect changes in position and/or relative orientation of the joints and/or suspension arm segments. For instance, a movement sensor 208 integrated into a joint of a suspension arm of the medical light 200 may detect movement based on movement of one or more of the segments connected to the joint.

The controller 204 is configured to determine that the medical light 200 is being repositioned based on signals from the one or more movement sensors 208. To determine the medical light 200 is being repositioned, the controller 204 may analyze signals from the one or more movement sensors 208 to determine one or more characteristics of the movement (such as an angular orientation or a translation of the medical light 200), or the controller 204 may analyze a digital measurement from a sensor module (not shown in figure) that includes one or more movement sensors 208 and an on-board processing unit that analyzes signals from the movement sensors 208 and outputs a digital measurement corresponding to a change in angle or a translation of the medical light 200.

The controller 204 can be configured to manage adjustment of one or more functions of the medical light 200 to prevent or reverse unintentional adjustments made by the user when using the handle assembly 210 to reposition the medical light 200 based on the controller 204 determining that the medical light 200 is being repositioned. Managing adjustment of one or more functions of the medical light 200 can include managing adjustments to one or more characteristics of the light sources 203 and/or the camera 213. To prevent inadvertent adjustment, the controller 204 can be configured to disable adjustment of at least one function of the medical light by the user input interfaces (e.g., the rotatable grip 209, buttons 216, capacitive sensors 218, rotatable rings 220, rotatable dials 214, and/or sliders 212) in response to determining that the medical light 200 is being repositioned, such that no adjustments can be made while the medical light 200 is being repositioned. Alternatively, to reverse inadvertent adjustment, the controller 204 can be configured to allow adjustments to be made while repositioning the medical light 200, but to reset (for example, upon detecting that the medical light 200 is finished being repositioned) the characteristics of the light sources 203 and/or the camera 213 to the setting that was used prior to the medical light 200 being repositioned. The controller 204 may be configured to account for inadvertent adjustments made prior to detecting that the medical light 200 is being repositioned, and/or adjustments made after detecting that repositioning of the medical light 200 is complete. For instance, the controller 204 may reverse any adjustments made within a predetermined period before detecting the medical light 200 is being repositioned and/or made within a predetermined period after detecting that the medical light 200 is finished being repositioned, or to disable adjustment during the predetermined period after determining the medical light 200 is finished being repositioned. The predetermined period may be, for example, a half a second, one second, two second, etc., such that any adjustments made within the predetermined period are reversed or disabled.

Optionally, the handle assembly 210 may include one or more indicators 222 (e.g., LEDs that can be illuminated) that provide feedback to a user regarding whether the user input interfaces of the handle assembly 210 can receive a user input. For instance, the indicators 222 may include LEDs that are illuminated in a red color while the user input interfaces of the handle assembly 210 are not enabled (e.g., during repositioning of the medical light 200) and thus cannot enact a user input that is received.

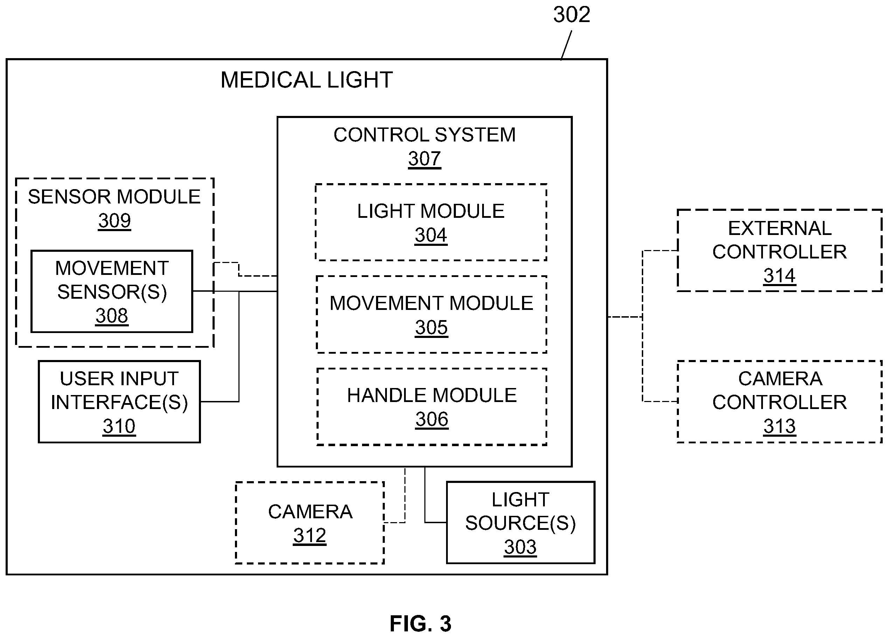

FIG. 3 shows a functional block diagram of a medical light 302. The medical light 302 can be configured as medical light 12 of FIG. 1A and/or medical light 200 of FIG. 2, and can include a control system 307, one or more movement sensors 308, one or more user input interfaces 310, and one or more light sources 303. The light sources 303 provide illumination light for illuminating a patient, and may be any suitable light emitter, such as an LED, an OLED, an incandescent bulb, a halogen bulb, a fluorescent bulb, etc., that can provide light for illuminating a patient.

The movement sensors 308 detect movement of the medical light 302. The movement sensors 308 can include one or more accelerometers, gyroscopes, position encoders, etc., that can detect translational movement, rotational movement, or both. The movement sensors 308 can output one or more signals to the control system 307, which the control system 307 can analyze to determine whether the medical light is being repositioned by a user. The signals output by the movement sensors can be, for example, acceleration signals from an accelerometer indicating the acceleration of the medical light 302 (e.g., whether the medical light is accelerating in a direction). The signals output by the movement sensors can be signals from a gyroscope indicating the angular rotation of the medical light 302. The signals output by the movement sensors may be position signals from an encoder indicating changes in position of segments and/or joints of a suspension arm, indicating that the medical light 302 is moving.

The movement sensors 308 may be integrated into a sensor module 309 that has a signal processor. The sensor module 309 may output a digital measurement indicative of the change in angle and/or position of the medical light to the control system 307. For instance, the sensor module 309 may analyze acceleration measurements from an accelerometer along one or more of the X, Y, and Z axes, and the sensor module 309 can output a digital measurement corresponding to the change in angle or position of the medical light relative to these axes to the control system 307.

Optionally, the sensor module 309 may process data from movement sensors 308 and determine one or more characteristics of the movement of the medical light, such as whether the medical light 302 is being repositioned or not. The sensor module 309 may then output one or more indications associated with the determined characteristics. For example, the sensor module 309 may output an indication that the medical light 302 is being repositioned or an indication that the medical light 302 is not being repositioned. By processing the sensor data within the sensor module 309, the communication and/or processing burden on the control system 307 may be reduced since the control system 307 need only obtain the one or more indications associated with the determined characteristics, rather than the stream(s) of sensor data.

As described above with respect to medical light 200 of FIG. 2, the user input interfaces 310 of medical light 302 can be part of a handle assembly and can include one or more of a rotatable grip, one or more buttons, one or more capacitive sensors, one or more rotatable rings, one or more rotatable dials, one or more sliders, or any combination of such features. The user input interfaces 310 can be part of a handle assembly of the medical light 302 (e.g., handle assembly 210 of FIG. 2) and receive user inputs for adjusting one or more characteristics of light provided by the light sources 303 of the medical light 302. For example, the user input interfaces 310 may receive user inputs for adjusting the intensity, spot size, color temperature, spectral distribution, and/or an on/off state of the light sources 303. Optionally, the medical light 302 may include a camera 312 located in a handle assembly of the medical light 302. The user input interfaces 310 may receive user inputs for adjusting one or more characteristics of the camera 312, such as the zoom, focus, white balance, and/or an on/off state of the camera 312.

The control system 307 can be configured to receive input information from the user input interfaces 310 to control one or more features of the medical light 302, such as the camera 312 and/or the light sources 303. For example, the control system 307 can receive input signals based on user inputs provided to the user input interfaces 310 and control the light sources 303 based on the provided user inputs. The control system 307 may receive input signals and then control the camera 312. The control system 307 may analyze the input signals to determine which characteristics of the light sources 303 or characteristics of the camera 312 should be adjusted based on information stored in a memory (e.g., memory 224 of FIG. 2) that maps certain handle assembly actuations to certain changes (e.g., press of a button controls on/off state).

The control system 307 can control the functions of the medical light 302 (e.g., the light sources 303 and/or camera 312) based on information received from an external controller 314 and/or a camera controller 313. The external controller 314 can be, for example, a medical light control panel such as a wall control for one or more medical lights in a room. A medical light control panel can include user input interfaces such as buttons, a touch screen, dials, etc., that a user can interact with to adjust one or more of the adjustable characteristics of light (e.g., the intensity, spot size, color temperature, spectral distribution, and/or an on/off status). Alternatively, the external controller 314 may be a computing system (such as a remote server) that manages settings for a plurality of medical lights. The camera controller 313 may be communicatively connected to the camera 312 such that the camera controller 313 can control the adjustable characteristics of the camera (e.g., zoom, focus, white balance, and/or an on/off status) directly. Alternatively, the camera controller 313 may communicate commands to control the camera 312 to the control system.

The control system 307 can be configured to determine that the medical light 302 is being repositioned based on signals from the movement sensors 308 of the medical light 302. The control system 307 may analyze a digital measurement from the sensor module 309 corresponding to a change in angle or position of the medical light 302. The control system 307 can compare the change in angle or position in this digital measurement to one or more predetermined thresholds to determine that the medical light 302 is being repositioned by a user.

Alternatively, the control system 307 may analyze the signals from the movement sensors 308 to determine one or more characteristics of movement of the medical light 302. The one or more characteristics of movement of the medical light 302 can be, for example, a change in angular orientation of the medical light 302 or a translation of the medical light 302. The one or more characteristics can be compared to one or more predetermined thresholds to determine that the medical light is being repositioned by a user. A change in angular orientation of the medical light 302 may be compared to an angular delta threshold, or a translation of the medical light 302 may be compared to a translational delta threshold. An exemplary angular delta threshold that may indicate the medical light is being repositioned can be any change in angular orientation of the medical light greater than 1 degree, 2 degrees, 5 degrees, 10 degrees, or 15 degrees. The predetermined angular delta threshold may be greater than 15 degrees or less than 1 degree. An exemplary translational delta threshold that may indicate the medical light is being repositioned can be any translation of the medical light greater than 5 mm, 10 mm, 15 mm, or 25 mm. The predetermined translational delta threshold may be greater than 25 mm or less than 5 mm.

For example, the control system 307 may analyze a signal from a gyroscope to determine an angular orientation of the medical light 302, which the control system 307 then compares to an angular delta threshold to determine whether the angular orientation indicates the medical light is being repositioned by a user. Similarly, the control system 307 may analyze a signal from an accelerometer to determine a translation of the medical light 302, and then compare the translation to a translational delta threshold to determine whether the translation indicates the medical light is being repositioned by a user. The control system 307 may analyze a signal from an encoder that indicates changes in positions of joints of a suspension arm of the medical light and compare those changes in positions of the joints to a translational delta threshold to determine whether the changes indicate the medical light 302 is being repositioned by a user.

When determining that the medical light is being repositioned, the control system 307 may be configured to distinguish between signals associated with repositioning of the medical light by a user and signals associated with other types of movement. For example, the control system 307 may associate a large acceleration with an inadvertent bump of the medical light and a lower acceleration with a purposeful repositioning of the medical light.
Optionally, the control system 307 utilizes one or more machine learning models to determine whether the medical light is being repositioned. The one or more machine learning models may have been trained to determine whether the medical light is being repositioned based on data from the movement sensors 308. During a training process, data from the movement sensors 308 may be collected while the medical light 302 is being repositioned, and the data may be labeled with labels associated with the desired predictions of the machine learning models. For example, the data may be labeled with a simple binary label of whether a given set of sensor data corresponds to repositioning or not repositioning such that the output of the machine learning model(s) is a prediction of whether the medical light is being repositioned or not. The one or more machine learning models may then be trained on the labeled training data.

As noted above, the sensor module 309 may be configured to process data from the movement sensors 308 and determine one or more characteristics of the movement of the medical light, such as whether the medical light 302 is being repositioned. As such, the sensor module 309 may be configured to perform any of the processing of the movement sensor data described above with respect to processing by the control system 307. In other words, rather than the control system 307 itself processing sensor data, such as to determine whether the medical light 302 is being repositioned or not, the control system 307 may simply query the sensor module 309 for the movement state of the medical light 302 and the sensor module 309 may provide the control system 307 with an indication of its determination of the movement state of the medical light 302. For example, the sensor module 309 may utilize one or more machine learning models to determine whether the medical light 302 is being repositioned (and/or any other characteristics of interest of movement of the medical light 302), as discussed above with respect to medical light 302.

The control system 307 can be configured to manage inadvertent adjustment of functions of the medical light (e.g., adjustments to the light sources 303 and/or camera 312) made while grasping a handle assembly of the medical light 302 to reposition the medical light 302. The control system 307 may prevent inadvertent adjustments entirely by disabling adjustment of characteristics of the light sources 303 and/or camera 312 in response to determining that the medical light 302 is being repositioned. For instance, the control system 307 may disable the user input interfaces 310 of the medical light 302 such that they cannot detect any user inputs while the medical light 302 is being repositioned. The control system 307 may alternatively ignore input information from the user input interfaces 310 by not controlling the light sources 303 and/or camera 312 based on input information received after determining that the medical light 302 is being repositioned. To disable adjustment while the medical light 302 is being repositioned, the control system 307 may be configured to not communicate any inputs purporting to adjust characteristics of the light sources 303 or the camera 312 to the external controller 314 and/or camera controller 313.

Alternatively, the control system 307 may allow inadvertent adjustments (e.g., adjustments made while the medical light is being repositioned) but then reset the characteristics of the medical light to a pre-movement setting after the medical light 302 is no longer being repositioned. For example, the control system 307 of the medical light 302 can control the light sources 303 to emit light according to a first setting that corresponds to values of one or more of the adjustable characteristics of the light and execute any adjustments to the one or more adjustable characteristics to a second setting upon receiving any input information to adjust the characteristics after determining the medical light is being repositioned, but then reset the one or more characteristics from the second setting back to the first setting in response to determining that the medical light is no longer being repositioned. For instance, if the medical light is initially providing light with a spot size of 10 cm but then while repositioning the medical light, the user unintentionally provides an input to a user input interface purporting to adjust the spot size to 20 cm, the control system 307 may reset the spot size of the light to 10 cm after the medical light is no longer being repositioned.

The control system 307 may save settings of one or more adjustable characteristics of the medical light periodically so that the medical light can be restored to a particular set of settings saved at a particular time. Restoring settings (or "resetting" the settings) to a particular set of settings saved at a particular time can be implemented to remove or effectively erase inadvertent changes made by a user when the user was preparing to reposition the medical light or finishing repositioning the medical light (e.g., in the process of gripping or releasing the handle of the medical light). Settings of the one or more adjustable characteristics can be saved periodically and the saved settings can be overwritten at intervals that ensure the saved settings are available for a predetermined time before movement of the medical light is detected. For instance, the predetermined time may be 1 second and the overwrite interval may be 2 seconds. Accordingly, a memory of the control system 307 may store settings according to a first time, time A, and settings according to a second time, time B, according to the settings of the medical light at time A and at time B. At a third time, time C, which is 1 second later than time B, the settings according to time A may be overwritten, with the control system 307 then storing the settings according to time B and to time C.

The control system 307 may be configured to reverse inadvertent adjustments made during a predetermined period before the medical light 302 was repositioned. As discussed above, in the configuration wherein the control system 307 allows adjustment while the medical light 302 is repositioned but then resets characteristics of the illumination light to a pre-movement setting, the control system 307 may not save any adjustments made while the light is repositioned and then reset the characteristics to the earliest saved pre-movement setting, or to a saved pre-movement setting. In the configuration wherein the control system 307 disables adjustment of functions of the medical light 302 while the medical light 302 is being repositioned, the control system 307 may reset the functions to the pre-movement settings when re-enabling adjustment after the medical light is no longer being repositioned.

The control system 307 may be configured to prevent inadvertent adjustments from being made during a predetermined period after the medical light 302 is finished being repositioned. In the configuration wherein the control system 307 disables adjustment of functions of the medical light in response to determining the medical light is being repositioned, the control system 307 can continue disabling adjustment of functions of the medical light for a predetermined period after determining that the medical light is no longer being repositioned (e.g., disable adjustment for a period after the medical light 302 is no longer being repositioned). If the control system 307 detects that the medical light begins moving during the predetermined period, this period may be reset. In the configuration wherein the control system allows adjustment while the medical light is repositioned but then resets characteristics of the illumination light to a pre-movement setting after the medical light 302 is no longer being repositioned, the control system 307 can wait a predetermined period after the medical light is no longer being repositioned to reset the characteristics of the medical light to the pre-movement settings.

The control system 307 can include one or more modules, such as light module 304, movement module 305, and handle module 306. One or more of the light module 304, the movement module 305, and the handle module 306 can be implemented as a software module of the control system 307, or as a separate controller. The light module 304 can be a software routine of the control system 307 that controls the light sources 303 of the medical light 302. For example, the light module 304 can control adjustments to the intensity, spot size, color temperature, spectral distribution, and/or an on/off state of the medical light 302. The handle module 306 can be a software routine of the control system 307 that analyzes inputs received via the user input interfaces 310 of a handle assembly of the medical light 302, determines which characteristics of the light sources 303 and/or the camera 312 should be altered and generates a command to control the light sources 303 and/or camera 312 accordingly. The command to control the light sources 303 can be executed by the light module 304, which then controls the light sources 303. The movement module 305 can be a software routine of the control system 307 that determines the medical light 302 is being repositioned. The movement module 305 can determine when the medical light is being repositioned and manage inadvertent adjustments by preventing adjustments to features (e.g., to the light sources 303 and/or camera 312) while the camera is being repositioned or reversing inadvertent adjustments after the camera is no longer being repositioned by resetting one or more settings to a pre-movement setting.

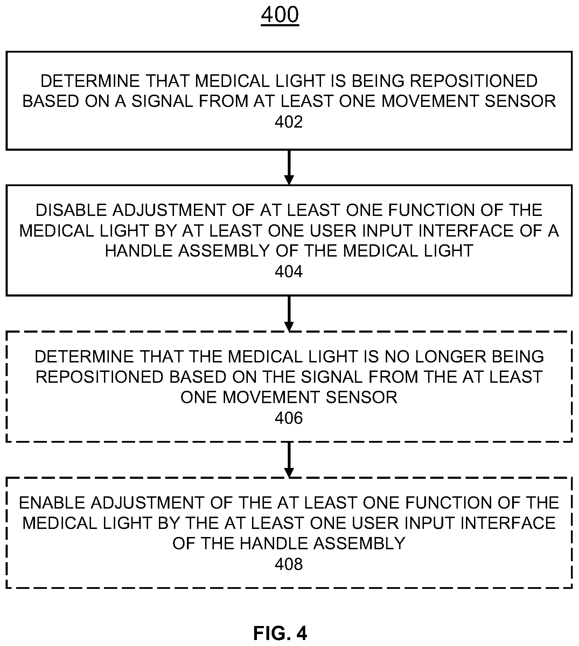

FIG. 4 is a block diagram of an exemplary method 400 for controlling at least one function of a medical light. Method 400 can be implemented to prevent inadvertent adjustment of functions of the medical light while the medical light is being repositioned. Method 400 may be performed, for example, by medical light 200 of FIG. 2 or medical light 302 of FIG. 3. In particular, method 400 may be performed by a controller of the medical light (such as controller 204 of medical light 200 of FIG. 2), via a control system (such as control system 307 of medical light 302 of FIG. 3), or via a controller communicatively connected to the medical light (e.g., external controller 314 and/or camera controller 313 of FIG. 3). Different aspects of method 400 may be implemented by different processing systems of the medical light. For example, with reference to FIG. 3, one or more aspects of method 400 may be implemented by sensor module 309, one or more aspects may be implemented by control system 307, and/or one or more aspects may be implemented by external controller 314.

At step 402, a determination that a medical light is being repositioned is made based on a signal from at least one movement sensor. A controller of the medical light may receive signals from one or more movement sensors, which may be located within a handle assembly of the medical light (e.g., movement sensor 208 of handle assembly 210 of FIG. 2), within a housing of the medical light (e.g., within housing 202 of medical light 200 of FIG. 2), on a suspension arm of the medical light (e.g., suspension arm 14 of FIG. 1A), or any combination of such sensors. The controller may analyze the signals from the movement sensors to determine one or more characteristics of movement of the medical light. The one or more characteristics of movement of the medical light can be, for example, a change in angular orientation of the medical light or a translation of the medical light. The controller can compare the one or more characteristics to one or more predetermined thresholds to determine that the medical light is being repositioned by a user. For example, a change in angular orientation of the medical light may be compared to an angular delta threshold, or a translation of the medical light may be compared to a translational delta threshold. Alternatively, the controller may analyze a digital measurement from a sensor module including at least one movement sensor that corresponds to a change in angle or position of the medical light. The controller may compare the change in angle or position of the medical light to one or more predetermined thresholds to determine that the medical light is being repositioned. Optionally, a sensor module, such as sensor module 309 of FIG. 3 may be configured to determine whether the medical light is being repositioned and may provide an indication of the determination to the controller. The controller may then determine that the medical light is being repositioned based on the indication received from the sensor module. Optionally, the controller may synthesize the determination of the sensor module 309 with other movement-related data that the controller receives to make the determination of whether the medical light is being repositioned. For example, the medical light may include multiple sensor modules, each configured to make its own determination of whether the medical light is being repositioned, and the controller may determine whether the medical light is being repositioned based on the output of the multiple sensors, such as determining that the medical light is being repositioned only if all sensor modules agree.
When determining that the medical light is being repositioned at step 402, the controller may be configured to distinguish between signals associated with repositioning of the medical light by a user and signals associated with other types of movement. In some examples, the controller implements a machine learning model trained to distinguish between signals associated with repositioning of the medical light by a user and signals associated with other types of movement. If the controller determines the signals from the one or more movement sensors are not associated with repositioning of the medical light by a user, the method 400 may cease, and the controller can continue operating the medical light as normal (i.e., controlling one or more light sources in response to user inputs via user input interfaces of the handle assembly).

At step 404, adjustment of at least one function of the medical light by at least one user input interface of the handle assembly is disabled in response to determining that the medical light is being repositioned at step 402. The functions of the medical light disabled at step 402 can include controlling the light sources of the medical light, and/or controlling a camera of a handle assembly of the medical light. In other words, disabling the light source function of the medical light can include disabling the control of the light sources of the medical light via the user input interfaces of the handle assembly such that one or more adjustable characteristics of light cannot be adjusted via the user input interfaces of the handle assembly. Similarly, disabling the camera function of the medical light can include disabling control of the camera of the medical light such that one or more adjustable characteristics of the camera cannot be adjusted via the user input interfaces.

Disabling adjustment of function(s) of the medical light at step 404 by the user input interfaces of the handle assembly can include disabling the user input interfaces of the medical light or disabling an input sensor connected to the user input interfaces such that no user inputs can be detected while the medical light is being repositioned. For instance, an input sensor can be disabled by the controller such that the input sensor cannot sense any inputs to the one or more user input interfaces (e.g., button 216, capacitive sensor 218, etc., and detected by a user input sensor such as input sensor 211) of the handle assembly. Alternatively, the controller can ignore any signals received from the input sensor after determining that the medical light is being repositioned by not controlling the function of the medical light based on input information received after determining that the medical light is being repositioned.

Disabling adjustment of at least one function at step 404 for a medical light that includes a camera in the handle assembly can include disabling communication of commands or input signals to an external controller communicatively connected to the camera. For instance, the controller of medical light can be communicatively connected to a camera controller to send camera adjustment commands to the camera controller based on user inputs to one or more user input interfaces of the handle assembly for adjusting one or more features of a camera, and upon determining that the medical light is being repositioned, the controller of the medical light can be configured to not communicate commands to the camera controller to adjust features of the camera.

The controller may disable adjustment of function(s) of the medical light at step 404 automatically in response to making the determination that the medical light is being repositioned at step 402 to prevent inadvertent adjustment of functions of the medical light while the medical light is being repositioned. Optionally, where the medical light includes an indicator for providing feedback to a user regarding a status of the user input interfaces (e.g., indicator 222 of FIG. 2), upon disabling adjustment of the at least one function of the medical light at step 404, the indicator can be controlled to indicate that the user input interfaces are disabled (e.g., turning on a red-colored LED).

Method 400 may include step 406, wherein a determination that the medical light is no longer being repositioned is made based on the signal from the at least one movement sensor. The controller may analyze the signals from the one or more movement sensors to determine whether the medical light is no longer being repositioned by a user. For example, the controller may analyze acceleration signals from an accelerometer to determine whether the acceleration profile from the accelerometer is associated with the medical light being stationary. The controller may analyze signals from a gyroscope associated with angular rotation to determine whether the angular rotation profile from the gyroscope is associated with the medical light being stationary. The controller may analyze a digital measurement from a signal processor that receives signals from one or more movement sensors that is indicative of the change in angle and/or position of the medical light to determine whether those changes are associated with the medical light being stationary.

Method 400 may include step 408, wherein adjustment of the at least one function of the medical light by the at least one user input interface of the handle assembly is enabled. Enabling the at least one user input interface at step 408 can include enabling the user input interfaces or input sensor that were disabled at step 404, resuming controlling function(s) of the medical light based on inputs received via the user input interfaces, and/or resuming to communicate input signals to a controller communicatively connected to the medical light. Adjustment of the at least one function may be enabled automatically in response to making the determination that the medical light is no longer being repositioned at step 406. Adjustment of the at least one function may be enabled after a predetermined period has elapsed after determining the medical light is no longer being repositioned. Optionally, where the medical light includes an indicator for providing feedback to a user regarding a status of the user input interfaces (e.g., indicator 222 of FIG. 2), step 408 can include controlling the indicator to indicate that the user input interfaces are no longer disabled (e.g., turning off a red-colored LED.)

Optionally, when enabling adjustment of the at least one function of the medical light at step 408, the method 400 can include a resetting step to account for inadvertent adjustments that were made to the at least one function immediately before determining that the medical light was being repositioned at step 402. Method 400 may involve resetting the at least one function of the medical light to the settings that the function(s) were operating at before the medical light was repositioned. When resetting functions of the medical light, the method 400 can access a memory with saved settings of the medical light and reset the settings to the function setting before determining the medical light was being repositioned. Settings of the one or more functions of the medical light may be saved periodically, with the saved settings being overwritten at specified intervals. For instance, settings may be saved periodically over a period of 1 second, with saved settings overwritten at 3 second intervals (such that 3 separate settings are saved at any given time).

As noted above, method 400 can be implemented to prevent inadvertent adjustment of functions of the medical light while the medical light is being repositioned. Alternatively, a medical light can be configured to allow adjustments to one or more functions of the medical light while the medical light is being repositioned, but then to reset those one or more functions to the setting at which they were operating prior to the medical light being repositioned by a user.

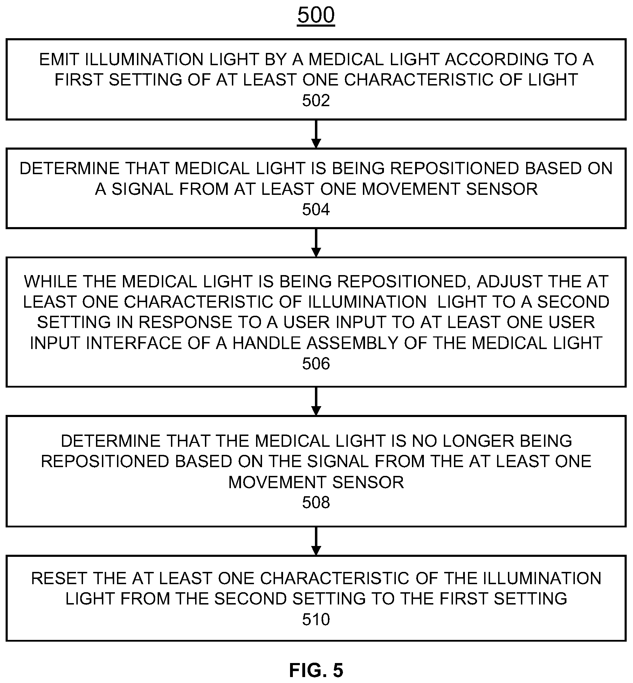

FIG. 5 is a block diagram of an exemplary method 500 for controlling at least one characteristic of illumination light provided by a medical light. Method 500 can be implemented to reverse inadvertent adjustment of characteristics of illumination light provided by the medical light made while the medical light was being repositioned and may be implemented in place of method 400. Method 500 may be implemented, for example, by medical light 200 of FIG. 2 or medical light 302 of FIG. 3. In particular, method 500 may be performed by a controller of the medical light (such as controller 204 of medical light 200 of FIG. 2), via a control system (such as control system 307 of medical light 302 of FIG. 3), or via a controller communicatively connected to the medical light (e.g., external controller 314 and/or camera controller 313 of FIG. 3).

At step 502, illumination light is emitted by a medical light according to a first setting of at least one characteristic of light. A setting of the medical light can correspond to values of one or more adjustable characteristics such as intensity, spot size, color temperature, spectral distribution, etc. Thus, providing light according to a first setting can include providing illumination light according to a combination of light characteristics. For instance, a first setting of at least one characteristic of light can correspond with a particular intensity and particular spot size. Optionally, each time the settings of one or more of the adjustable characteristics of the medical light are altered, the current settings of the medical light can be saved as an updated setting (e.g., a first setting) in a memory of the medical light (such as memory 224 of medical light 200 of FIG. 2). The first setting of characteristic(s) of light may correspond to a global setting for a plurality of medical lights. For instance, a plurality of medical lights may each be operated according to the same global setting of characteristics.

At step 504, a determination that the medical light is being repositioned is made based on a signal from at least one movement sensor. Step 504 can be the same as step 402 of method 400. Accordingly, details of step 504 are omitted for brevity.

The setting at which the medical light is currently operated (e.g., the first setting of step 502) may be saved automatically in response to determining that the medical light is being repositioned at step 504. Optionally, the setting at which the medical light is currently operating can be saved prior to determining that the medical light is being repositioned at step 504. For example, each time the settings of one or more adjustable characteristics of the medical light are altered, the settings may be saved such that when a determination is made that the medical light is being repositioned, the settings of the functions/characteristics of the medical light prior to the repositioning have already been stored in the memory. The settings of one or more adjustable characteristics of the medical light may be saved periodically so that the medical light can be restored to a particular set of settings saved at a particular time. For example, the settings may be restored to a predetermined period of time prior to the medical light being moved so that inadvertent adjustments made as the user was preparing to move the medical light (e.g., in the process of gripping the handle) can be reversed. Settings may be saved periodically and overwritten at intervals that ensure that saved settings are available for a predetermined time prior to movement of the medical light being detected. For example, the predetermined time may be 1 second, and the overwrite interval may be 2 seconds. Optionally, to account for inadvertent adjustments both before and after the medical light is repositioned, adjustments made during the predetermined period before and/or after the medical light is repositioned may not be saved.

At step 506, at least one characteristic of illumination light is adjusted in response to a user input to at least one user input interface of a handle assembly of the medical light while the medical light is being repositioned. For example, a user may inadvertently twist a grip of the handle assembly and the medical light may respond by adjusting one or more characteristics of the light just as if the user had not been repositioning the medical light. Hence, in step 506 the method enables adjustment of a setting of at least one function while the medical light is being repositioned.

At step 508, a determination that the medical light is no longer being repositioned is made based on the signal from the at least one movement sensor. The controller may analyze the signals from the one or more movement sensors to determine whether the medical light is no longer being repositioned by a user. For example, the controller may analyze acceleration signals from an accelerometer to determine whether the acceleration profile from the accelerometer is associated with the medical light being stationary. The controller may analyze signals from a gyroscope associated with angular rotation to determine whether the angular rotation profile from the gyroscope is associated with the medical light being stationary. The controller may analyze a digital measurement from a signal processor that receives signals from one or more movement sensors that is indicative of the change in angle and/or position of the medical light to determine whether those changes are associated with the medical light being stationary.

At step 510, the at least one characteristic of the illumination light is reset from the setting that the at least one characteristic was adjusted to back to the first setting. As noted above, the first setting corresponds to the setting at which the medical light was being operated prior to being repositioned. Accordingly, when resetting the setting of the medical light from the second setting (updated while the medical light was being repositioned) to the first setting (the setting before repositioning), step 510 effectively erases any updates to characteristics of the medical light that were made while the light was being repositioned, which may have inadvertently been affected while the user was grasping the handle of the medical light to reposition the medical light. The at least one characteristic of the illumination light may be reset in response to determining that the medical light is no longer being repositioned at step 508. Hence, step 508 may provide that, in response to determining that the medical light is no longer being repositioned, the at least one function of the light is returned back to its setting prior to the medical light being repositioned. The at least one characteristic of the illumination light may instead be reset after a predetermined period has elapsed after determining that the medical light is no longer being repositioned.

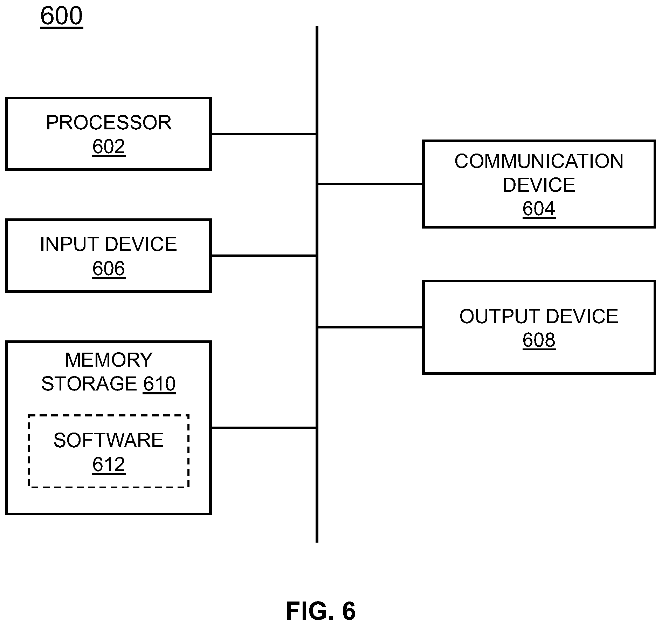

FIG. 6 illustrates an exemplary computing device 600. Device 600 can be a controller such as any of controller 204, handle controller 206, control system 307, external controller 314 and/or camera controller 313. Device 600 can be a host computer connected to a network. Device 600 can be a client computer or a server. As shown in FIG. 6, device 600 can be any suitable type of microprocessor-based device, such as a personal computer, workstation, server, or handheld computing device (portable electronic device) such as a phone or tablet. The device can include, for example, one or more of processors 602, input device 606, output device 608, storage 610, and communication device 604. Input device 606 and output device 608 can generally correspond to those described above and can either be connectable or integrated with the computer.

Input device 606 can be any suitable device that provides input, such as a touch screen, keyboard or keypad, mouse, or voice-recognition device. Output device 608 can be any suitable device that provides output, such as a touch screen, haptics device, or speaker. The input device 606 can receive user inputs for controlling at least one characteristic of light provided by a medical light and may be located on a medical light (e.g., any of user input interfaces 212, 214, 216, 218, 220, and handle assembly 210 of FIG. 2) or externally from a medical light (such as on a user interface of external controller 314 and/or camera controller 313 of FIG. 3).

Storage 610 can be any suitable device that provides storage, such as an electrical, magnetic, or optical memory, including a RAM, cache, hard drive, or removable storage disk. Communication device 604 can include any suitable device capable of transmitting and receiving signals over a network, such as a network interface chip or device. The components of the computer can be connected in any suitable manner, such as via a physical bus or wirelessly.

Software 612, which can be stored in storage 610 and executed by processor 602, can include, for example, the programming that embodies the functionality of the present disclosure (e.g., as embodied in the devices as described above), such as for implementing one or more steps of method 400 of FIG. 4 and/or method 500 of FIG. 5.

Software 612 can also be stored and/or transported within any non-transitory computer-readable storage medium for use by or in connection with an instruction execution system, apparatus, or device, such as those described above, that can fetch instructions associated with the software from the instruction execution system, apparatus, or device and execute the instructions. In the context of this disclosure, a computer-readable storage medium can be any medium, such as storage 610, that can contain or store programming for use by or in connection with an instruction execution system, apparatus, or device.

Software 612 can also be propagated within any transport medium for use by or in connection with an instruction execution system, apparatus, or device, such as those described above, that can fetch instructions associated with the software from the instruction execution system, apparatus, or device and execute the instructions. In the context of this disclosure, a transport medium can be any medium that can communicate, propagate, or transport programming for use by or in connection with an instruction execution system, apparatus, or device. The transport readable medium can include, but is not limited to, an electronic, magnetic, optical, electromagnetic, or infrared wired or wireless propagation medium.

Device 600 may be connected to a network, which can be any suitable type of interconnected communication system. The network can implement any suitable communications protocol and can be secured by any suitable security protocol. The network can comprise network links of any suitable arrangement that can implement the transmission and reception of network signals, such as wireless network connections, T1 or T3 lines, cable networks, DSL, or telephone lines.

Device 600 can implement any operating system suitable for operating on the network. Software 612 can be written in any suitable programming language, such as C, C++, Java, or Python. In various embodiments, application software embodying the functionality of the present disclosure can be deployed in different configurations, such as in a client/server arrangement or through a Web browser as a Web-based application or Web service, for example.

Accordingly, described herein are systems, devices, and methods that prevent or reverse inadvertent adjustment of characteristics of light made using a handle assembly of the medical light while the medical light is being repositioned via that handle assembly. To prevent inadvertent adjustment, the medical light can be configured to disable adjustment of characteristics of light while the medical light is being repositioned, which may be determined based on a signal from a movement sensor. To reverse inadvertent adjustments, the medical light can be configured to reset a setting of characteristics of the medical light to a first setting that corresponds to the setting of the medical light prior to being repositioned after the medical light has been repositioned.

## Claims

1. A method for controlling a medical light (12, 200, 302), the method comprising:
determining that the medical light is being repositioned based on a signal from at least one movement sensor (208); and
in response to determining that the medical light is being repositioned:
disabling adjustment of at least one function of the medical light by at least one user input interface (310) of a handle assembly (10, 210) of the medical light, comprising while the medical light is being repositioned, receiving a user input via the at least one user input interface to adjust the at least one function of the medical light (12, 200); and
ignoring the user input to adjust the at least one function of the medical light; or
enabling adjustment of a setting of at least one function while the medical light (12, 200, 302) is being repositioned comprising receiving a user input via the at least one user input interface (310) to adjust the at least one function of the medical light,
and in response to determining that the medical light is no longer being repositioned return the at least one function of the light back to its setting prior to the medical light being repositioned.

2. The method of claim 1, wherein the at least one function of the medical light (12, 200, 302) comprises providing illumination light according to at least one setting, and disabling adjustment of the at least one function comprises disabling adjustment of the at least one setting, wherein the at least one setting e.g. comprises an intensity of the illumination light provided by the medical light (12, 200, 302), a spot size of the illumination light, a color temperature of the illumination light, or a spectral distribution of the illumination light.

3. The method of claim 1 or 2, comprising saving the setting of at least one function in a memory (610) prior to determining that the medical light (12, 200, 302) is being repositioned and/or in response to determining that the medical light is being repositioned.

4. The method of any of claims 1-3, comprising:
determining that the medical light (12, 200, 302) is no longer being repositioned based on the signal from the at least one movement sensor (208); and in response to determining that the medical light is no longer being repositioned, enabling adjustment of the at least one function of the medical light by the at least one user input interface of the handle assembly (10, 210).

5. The method of any of claims 1-4, wherein the signal from the at least one movement sensor (208) is associated with a change in angle and/or translation of the medical light (12, 200, 302), wherein determining that the medical light is being repositioned optionally comprises determining that the change in angle and/or translation of the medical light exceeds one or more predetermined thresholds.

6. The method of claim **1,** wherein the user input comprises at least one of: a twist of a handle (209) of the handle assembly (10, 210), a press of a button (216) of the handle assembly, a touch of a capacitive sensor (218) of the handle assembly, a rotation of a dial (214), a slide of a slider knob (212), and a rotation of a ring (220) of the handle assembly.

7. The method of any of claims 1-6, wherein the at least one function comprises a characteristic of a camera (213) of the medical light (12, 200, 302), wherein optionally the camera (213) is controlled by an external camera controller (313) and disabling adjustment of the at least one function comprises disabling communication of commands to the external camera controller.

8. A medical light (12, 200, 302) comprising:
a handle assembly (10, 210) configured for grasping by a user for moving the medical light, the handle assembly comprising at least one user input interface (310) configured to receive inputs from the user for adjusting at least one function of the medical light;
at least one movement sensor (208, 308) configured to detect movement of the medical light (12, 200, 302); and
a controller (204, 307, 600) comprising one or more processors (602), memory (610), and one or more programs (612) stored in the memory for execution by the one or more processors, wherein the one or more programs include instructions that, when executed by the one or more processors cause the controller to:
determine that the medical light (12, 200, 302) is being repositioned based on a signal from the at least one movement sensor (208, 308); and in response to determining that the medical light is being repositioned:
disable adjustment of the at least one function of the medical light by the at least one user input interface (310) of the handle assembly (10, 210) comprising while the medical light is being repositioned, receiving a user input via the at least one user input interface to adjust the at least one function of the medical light; and ignoring the user input to adjust the at least one function of the medical light; or
enable adjustment of a setting of the at least one function while the medical light is being repositioned comprising receiving a user input via the at least one user input interface (310) to adjust the at least one function of the medical light, and in response to determining that the medical light is no longer being repositioned return the at least one function of the light back to its setting prior to the medical light being repositioned.

9. The medical light (12, 200, 302) of claim 8, wherein the controller (204, 307, 600) is located in the handle assembly (10, 210), and/or in a housing (202) of the medical light.

10. The medical light (12, 200, 302) of claim 8 or 9, wherein the medical light comprises an arm (14) that comprises the at least one movement sensor (208, 308), and/or wherein the at least one movement sensor comprises an accelerometer, a gyroscope, or a position encoder.

11. The medical light (12, 200, 302) of any of claims 8-10, wherein the at least one user input interface (310) comprises one of a twistable handle (209), at least one button (216), at least one touch sensor (218), at least one rotatable dial (214), at least one slider knob (212), and at least one rotatable ring (220).

12. The medical light (12, 200, 302) of any of claims 8-11, wherein the at least one setting comprises an intensity of the illumination light provided by the medical light, a spot size of the illumination light, a color temperature of the illumination light, or a spectral distribution of the illumination light.

13. The medical light (12, 200, 302) of any of claims 8-12, wherein the one or more programs (612) include instructions that, when executed by the one or more processors cause the controller (204, 307, 600) to:
determine that the medical light is no longer being repositioned based on the signal from the at least one movement sensor (208, 308); and
in response to determining that the medical light is no longer being repositioned, enable adjustment of the at least one function of the medical light by the at least one user input interface (310) of the handle assembly (10, 210).

14. The medical light (12, 200, 302) of any of claims 8-13, wherein the signal from the at least one movement sensor (208, 308) is associated with a change in angle and/or position of the medical light, wherein optionally determining that the medical light is being repositioned comprises determining that the change in angle and/or position of the medical light exceeds one or more predetermined thresholds.

15. The medical light (12, 200, 302) of any of claims 8-14, wherein the medical light comprises a camera (213), wherein optionally the camera is controlled by an external camera controller (313), and disabling adjustment of the at least one function comprises disabling communication of commands to the external camera controller.

16. A computer program product comprising one or more software code portions for controlling at least one characteristic of illumination light provided by a medical light (12, 200, 302), the one or more software code portions comprising instructions which, when executed by a controller (204, 307, 600) of a medical light, cause the controller to perform the method of any of claims 1-7.

## Patentansprüche

1. Verfahren zum Steuern einer medizinischen Leuchte (12, 200, 302), wobei das Verfahren Folgendes umfasst:
Bestimmen, dass die medizinische Leuchte neu positioniert wird, basierend auf einem Signal von mindestens einem Bewegungssensor (208); und
als Reaktion auf das Bestimmen, dass die medizinische Leuchte neu positioniert wird: Deaktivieren der Anpassung von mindestens einer Funktion der medizinischen Leuchte durch mindestens eine Benutzereingabeschnittstelle (310) einer Griffbaugruppe (10, 210) der medizinischen Leuchte, umfassend, während die medizinische Leuchte neu positioniert wird, das Empfangen einer Benutzereingabe über die mindestens eine Benutzereingabeschnittstelle zum Anpassen der mindestens einen Funktion der medizinischen Leuchte (12, 200); und
Ignorieren der Benutzereingabe zum Anpassen der mindestens einen Funktion der medizinischen Leuchte; oder
Ermöglichen der Anpassung von einer Einstellung von mindestens einer Funktion, während die medizinische Leuchte (12, 200, 302) neu positioniert wird, umfassend das Empfangen einer Benutzereingabe über die mindestens eine Benutzereingabeschnittstelle (310) zum Anpassen der mindestens einen Funktion der medizinischen Leuchte,
und als Reaktion auf das Bestimmen, dass die medizinische Leuchte nicht länger neu positioniert wird, Zurücksetzen der mindestens einen Funktion der Leuchte auf ihre Einstellung vor der Neupositionierung der medizinischen Leuchte.

2. Verfahren nach Anspruch 1, wobei die mindestens eine Funktion der medizinischen Leuchte (12, 200, 302) das Bereitstellen von Beleuchtungslicht gemäß mindestens einer Einstellung umfasst, und das Deaktivieren der Anpassung der mindestens einen Funktion das Deaktivieren der Anpassung der mindestens einen Einstellung umfasst, wobei die mindestens eine Einstellung z. B. eine Intensität des von der medizinischen Leuchte (12, 200, 302) bereitgestellten Beleuchtungslichts, eine Spotgröße des Beleuchtungslichts, eine Farbtemperatur des Beleuchtungslichts oder eine Spektralverteilung des Beleuchtungslichts umfasst.

3. Verfahren nach Anspruch 1 oder 2, umfassend das Speichern der Einstellung von mindestens einer Funktion in einem Speicher (610), vor dem Bestimmen, dass die medizinische Leuchte (12, 200, 302) neu positioniert wird, und/oder als Reaktion auf das Bestimmen, dass die medizinische Leuchte neu positioniert wird.

4. Verfahren nach einem der Ansprüche 1 bis 3, umfassend:
Bestimmen, dass die medizinische Leuchte (12, 200, 302) nicht länger neu positioniert wird, basierend auf dem Signal von dem mindestens einen Bewegungssensor (208); und
als Reaktion auf das Bestimmen, dass die medizinische Leuchte nicht länger neu positioniert wird, Ermöglichen der Anpassung der mindestens einen Funktion der medizinischen Leuchte durch die mindestens eine Benutzereingabeschnittstelle der Griffbaugruppe (10, 210).

5. Verfahren nach einem der Ansprüche 1 bis 4, wobei das Signal von dem mindestens einen Bewegungssensor (208) einer Änderung von Winkel und/oder Translation der medizinischen Leuchte (12, 200, 302) zugeordnet ist, wobei das Bestimmen, dass die medizinische Leuchte neu positioniert wird, optional das Bestimmen umfasst, dass die Änderung von Winkel und/oder Translation der medizinischen Leuchte einen oder mehrere vorbestimmte Schwellenwerte überschreitet.

6. Verfahren nach Anspruch 1, wobei die Benutzereingabe mindestens eines von Folgendem umfasst: eine Drehung eines Griffs (209) der Griffbaugruppe (10, 210), eine Betätigung einer Taste (216) der Griffbaugruppe, eine Berührung eines kapazitiven Sensors (218) der Griffbaugruppe, eine Rotation eines Wählrads (214), ein Schieben eines Schiebeknopfs (212) und eine Rotation eines Rings (220) der Griffbaugruppe.

7. Verfahren nach einem der Ansprüche 1 bis 6, wobei die mindestens eine Funktion eine Eigenschaft einer Kamera (213) der medizinischen Leuchte (12, 200, 302) umfasst, wobei optional die Kamera (213) durch eine externe Kamerasteuerung (313) gesteuert wird und das Deaktivieren der Anpassung der mindestens einen Funktion das Deaktivieren der Kommunikation von Befehlen an die externe Kamerasteuerung umfasst.

8. Medizinische Leuchte (12, 200, 302) umfassend:
eine Griffbaugruppe (10, 210), die dazu konfiguriert ist, von einem Benutzer zum Bewegen der medizinischen Leuchte ergriffen zu werden, wobei die Griffbaugruppe mindestens eine Benutzereingabeschnittstelle (310) umfasst, die dazu konfiguriert ist, Eingaben von dem Benutzer zum Anpassen von mindestens einer Funktion der medizinischen Leuchte zu empfangen;
mindestens einen Bewegungssensor (208, 308), der dazu konfiguriert ist, Bewegungen der medizinischen Leuchte (12, 200, 302) zu erfassen; und
eine Steuerung (204, 307, 600), umfassend einen oder mehrere Prozessoren (602), einen Speicher (610) und ein oder mehrere Programme (612), die in dem Speicher zur Ausführung durch den einen oder die mehreren Prozessoren gespeichert sind, wobei das eine oder die mehreren Programme Anweisungen einschließen, die bei Ausführung durch den einen oder die mehreren Prozessoren die Steuerung zu Folgendem veranlassen:
Bestimmen, dass die medizinische Leuchte (12, 200, 302) neu positioniert wird, basierend auf einem Signal von dem mindestens einen Bewegungssensor (208, 308); und als Reaktion auf das Bestimmen, dass die medizinische Leuchte neu positioniert wird:
Deaktivieren der Anpassung der mindestens einen Funktion der medizinischen Leuchte durch die mindestens eine Benutzereingabeschnittstelle (310) der Griffbaugruppe (10, 210), umfassend, während die medizinische Leuchte neu positioniert wird, das Empfangen einer Benutzereingabe über die mindestens eine Benutzereingabeschnittstelle zum Anpassen der mindestens einen Funktion der medizinischen Leuchte; und Ignorieren der Benutzereingabe zum Anpassen der mindestens einen Funktion der medizinischen Leuchte; oder
Ermöglichen der Anpassung von einer Einstellung der mindestens einen Funktion, während die medizinische Leuchte neu positioniert wird, umfassend das Empfangen einer Benutzereingabe über die mindestens eine Benutzereingabeschnittstelle (310) zum Anpassen der mindestens einen Funktion der medizinischen Leuchte, und als Reaktion auf das Bestimmen, dass die medizinische Leuchte nicht länger neu positioniert wird, Zurücksetzen der mindestens einen Funktion der Leuchte auf ihre Einstellung vor der Neupositionierung der medizinischen Leuchte.

9. Medizinische Leuchte (12, 200, 302) nach Anspruch 8, wobei sich die Steuerung (204, 307, 600) in der Griffbaugruppe (10, 210) und/oder in einem Gehäuse (202) der medizinischen Leuchte befindet.

10. Medizinische Leuchte (12, 200, 302) nach Anspruch 8 oder 9, wobei die medizinische Leuchte einen Arm (14) umfasst, der den mindestens einen Bewegungssensor (208, 308) umfasst, und/oder wobei der mindestens eine Bewegungssensor einen Beschleunigungsmesser, ein Gyroskop oder einen Positionsgeber umfasst.

11. Medizinische Leuchte (12, 200, 302) nach einem der Ansprüche 8 bis 10, wobei die mindestens eine Benutzereingabeschnittstelle (310) eines von einem drehbaren Griff (209), mindestens einer Taste (216), mindestens einem Berührungssensor (218), mindestens einem drehbaren Wählrad (214), mindestens einem Schiebeknopf (212) und mindestens einem drehbaren Ring (220) umfasst.

12. Medizinische Leuchte (12, 200, 302) nach einem der Ansprüche 8 bis 11, wobei die mindestens eine Einstellung eine Intensität des von der medizinischen Leuchte bereitgestellten Beleuchtungslichts, eine Spotgröße des Beleuchtungslichts, eine Farbtemperatur des Beleuchtungslichts oder eine Spektralverteilung des Beleuchtungslichts umfasst.

13. Medizinische Leuchte (12, 200, 302) nach einem der Ansprüche 8 bis 12, wobei das eine oder die mehreren Programme (612) Anweisungen einschließen, die bei Ausführung durch den einen oder die mehreren Prozessoren die Steuerung (204, 307, 600) zu Folgendem veranlassen:
Bestimmen, dass die medizinische Leuchte nicht länger neu positioniert wird, basierend auf dem Signal von dem mindestens einen Bewegungssensor (208, 308); und
als Reaktion auf das Bestimmen, dass die medizinische Leuchte nicht länger neu positioniert wird, Ermöglichen der Anpassung der mindestens einen Funktion der medizinischen Leuchte durch die mindestens eine Benutzereingabeschnittstelle (310) der Griffbaugruppe (10, 210).

14. Medizinische Leuchte (12, 200, 302) nach einem der Ansprüche 8 bis 13, wobei das Signal von dem mindestens einen Bewegungssensor (208, 308) einer Änderung von Winkel und/oder Position der medizinischen Leuchte zugeordnet ist, wobei optional das Bestimmen, dass die medizinische Leuchte neu positioniert wird, das Bestimmen umfasst, dass die Änderung von Winkel und/oder Position der medizinischen Leuchte einen oder mehrere vorbestimmte Schwellenwerte überschreitet.

15. Medizinische Leuchte (12, 200, 302) nach einem der Ansprüche 8 bis 14, wobei die medizinische Leuchte eine Kamera (213) umfasst, wobei optional die Kamera durch eine externe Kamerasteuerung (313) gesteuert wird, und das Deaktivieren der Anpassung der mindestens einen Funktion das Deaktivieren der Kommunikation von Befehlen an die externe Kamerasteuerung umfasst.

16. Computerprogrammprodukt, umfassend einen oder mehrere Softwarecodeabschnitte zum Steuern von mindestens einer Eigenschaft des von einer medizinischen Leuchte (12, 200, 302) bereitgestellten Beleuchtungslichts, wobei der eine oder die mehreren Softwarecodeabschnitte Anweisungen umfassen, die, bei Ausführung durch eine Steuerung (204, 307, 600) einer medizinischen Leuchte, die Steuerung dazu veranlassen, das Verfahren nach einem der Ansprüche 1 bis 7 durchzuführen.

## Revendications

1. Procédé pour la commande d'une lampe médicale (12, 200, 302), le procédé comprenant :
la détermination que la lampe médicale est en train d'être repositionnée sur la base d'un signal provenant d'au moins un capteur de mouvement (208) ; et
en réponse à la détermination que la lampe médicale est en train d'être repositionnée :
le fait d'empêcher l'ajustement d'au moins une fonction de la lampe médicale par au moins une interface d'entrée d'utilisateur (310) d'un ensemble poignée (10, 210) de la lampe médicale, comprenant, alors que la lampe médicale est en train d'être repositionnée, la réception d'une entrée d'utilisateur via l'au moins une interface d'entrée d'utilisateur d'ajuster l'au moins une fonction de la lampe médicale (12, 200) ; et
le fait d'ignorer l'entrée d'utilisateur d'ajuster l'au moins une fonction de la lampe médicale ; ou
le fait de permettre l'ajustement d'un réglage d'au moins une fonction alors que la lampe médicale (12, 200, 302) est en train d'être repositionnée, comprenant la réception d'une entrée d'utilisateur via l'au moins une interface d'entrée d'utilisateur (310) d'ajuster l'au moins une fonction de la lampe médicale,
et en réponse à la détermination que la lampe médicale n'est plus en train d'être repositionnée, ramener l'au moins une fonction de la lampe dans son réglage avant que la lampe médicale ne soit repositionnée.

2. Procédé selon la revendication 1, dans lequel l'au moins une fonction de la lampe médicale (12, 200, 302) comprend la fourniture d'une lumière d'éclairage selon au moins un réglage, et le fait d'empêcher l'ajustement de l'au moins une fonction comprend le fait d'empêcher l'ajustement de l'au moins un réglage, dans lequel l'au moins un réglage, par ex., comprend une intensité de la lumière d'éclairage fournie par la lampe médicale (12, 200, 302), une taille de tache de la lumière d'éclairage, une température de couleur de la lumière d'éclairage, ou une distribution spectrale de la lumière d'éclairage.

3. Procédé selon la revendication 1 ou 2, comprenant la sauvegarde du réglage d'au moins une fonction dans une mémoire (610) avant la détermination que la lampe médicale (12, 200, 302) est en train d'être repositionnée et/ou en réponse à la détermination que la lampe médicale est en train d'être repositionnée.

4. Procédé selon l'une quelconque des revendications 1 à 3, comprenant :
la détermination que la lampe médicale (12, 200, 302) n'est plus en train d'être repositionnée sur la base du signal provenant de l'au moins un capteur de mouvement (208) ; et en réponse à la détermination que la lampe médicale n'est plus en train d'être repositionnée, le fait de permettre l'ajustement de l'au moins une fonction de la lampe médicale par l'au moins une interface d'entrée d'utilisateur de l'ensemble poignée (10, 210).

5. Procédé selon l'une quelconque des revendications 1 à 4, dans lequel le signal provenant de l'au moins un capteur de mouvement (208) est associé à un changement d'angle et/ou de translation de la lampe médicale (12, 200, 302), dans lequel la détermination que la lampe médicale est en train d'être repositionnée comprend facultativement la détermination que le changement d'angle et/ou de translation de la lampe médicale dépasse un ou plusieurs seuils prédéterminés.

6. Procédé selon la revendication 1, dans lequel l'entrée d'utilisateur comprend au moins l'un parmi : une torsion d'une poignée (209) de l'ensemble poignée (10, 210), une pression sur un bouton (216) de l'ensemble poignée, un toucher d'un capteur capacitif (218) de l'ensemble poignée, une rotation d'un cadran (214), un glissement d'un curseur de glissière (212), et une rotation d'une bague (220) de l'ensemble poignée.

7. Procédé selon l'une quelconque des revendications 1 à 6, dans lequel l'au moins une fonction comprend une caractéristique d'un appareil de prise de vues (213) de la lampe médicale (12, 200, 302), dans lequel facultativement l'appareil de prise de vues (213) est commandé par un dispositif de commande d'appareil de prise de vues externe (313) et le fait d'empêcher l'ajustement de l'au moins une fonction comprend le fait d'empêcher la communication de consignes au dispositif de commande d'appareil de prise de vues externe.

8. Lampe médicale (12, 200, 302) comprenant :
un ensemble poignée (10, 210) configuré pour une saisie par un utilisateur pour le déplacement de la lampe médicale, l'ensemble poignée comprenant au moins une interface d'entrée d'utilisateur (310) configurée pour recevoir des entrées provenant de l'utilisateur pour l'ajustement d'au moins une fonction de la lampe médicale ;
au moins un capteur de mouvement (208, 308) configuré pour détecter un mouvement de la lampe médicale (12, 200, 302) ; et
un dispositif de commande (204, 307, 600) comprenant un ou plusieurs processeurs (602), de la mémoire (610), et un ou plusieurs programmes (612) stockés dans la mémoire pour une exécution par les un ou plusieurs processeurs, dans laquelle les un ou plusieurs programmes incluent des instructions qui, lors de leur exécution par les un ou plusieurs processeurs, amènent le dispositif de commande à :
déterminer que la lampe médicale (12, 200, 302) est en train d'être repositionnée sur la base d'un signal provenant de l'au moins un capteur de mouvement (208, 308) ; et en réponse à la détermination que la lampe médicale est en train d'être repositionnée :
empêcher l'ajustement de l'au moins une fonction de la lampe médicale par l'au moins une interface d'entrée d'utilisateur (310) de l'ensemble poignée (10, 210), comprenant, alors que la lampe médicale est en train d'être repositionnée, la réception d'une entrée d'utilisateur via l'au moins une interface d'entrée d'utilisateur d'ajuster l'au moins une fonction de la lampe médicale ; et le fait d'ignorer l'entrée d'utilisateur d'ajuster l'au moins une fonction de la lampe médicale ; ou
permettre l'ajustement d'un réglage de l'au moins une fonction alors que la lampe médicale est en train d'être repositionnée, comprenant la réception d'une entrée d'utilisateur via l'au moins une interface d'entrée d'utilisateur (310) d'ajuster l'au moins une fonction de la lampe médicale, et en réponse à la détermination que la lampe médicale n'est plus en train d'être repositionnée, ramener l'au moins une fonction de la lampe dans son réglage avant que la lampe médicale ne soit repositionnée.

9. Lampe médicale (12, 200, 302) selon la revendication 8, dans laquelle le dispositif de commande (204, 307, 600) est situé dans l'ensemble poignée (10, 210), et/ou dans un boîtier (202) de la lampe médicale.

10. Lampe médicale (12, 200, 302) selon la revendication 8 ou 9, dans laquelle la lampe médicale comprend un bras (14) qui comprend l'au moins un capteur de mouvement (208, 308), et/ou dans laquelle l'au moins un capteur de mouvement comprend un accéléromètre, un gyroscope ou un codeur de position.

11. Lampe médicale (12, 200, 302) selon l'une quelconque des revendications 8 à 10, dans laquelle l'au moins une interface d'entrée d'utilisateur (310) comprend l'un parmi une poignée apte à subir une torsion (209), au moins un bouton (216), au moins un capteur tactile (218), au moins un cadran rotatif (214), au moins un curseur de glissière (212), et au moins un bague rotative (220).

12. Lampe médicale (12, 200, 302) selon l'une quelconque des revendications 8 à 11, dans laquelle l'au moins un réglage comprend une intensité de la lumière d'éclairage fournie par la lampe médicale, une taille de tache de la lumière d'éclairage, une température de couleur de la lumière d'éclairage, ou une distribution spectrale de la lumière d'éclairage.

13. Lampe médicale (12, 200, 302) selon l'une quelconque des revendications 8 à 12, dans laquelle les un ou plusieurs programmes (612) incluent des instructions qui, lors de leur exécution par les un ou plusieurs processeurs, amènent le dispositif de commande (204, 307, 600) à :
déterminer que la lampe médicale n'est plus en train d'être repositionnée sur la base du signal provenant de l'au moins un capteur de mouvement (208, 308) ; et
en réponse à la détermination que la lampe médicale n'est plus en train d'être repositionnée, permettre l'ajustement de l'au moins une fonction de la lampe médicale par l'au moins une interface d'entrée d'utilisateur (310) de l'ensemble poignée (10, 210).

14. Lampe médicale (12, 200, 302) selon l'une quelconque des revendications 8 à 13, dans laquelle le signal provenant de l'au moins un capteur de mouvement (208, 308) est associé à un changement d'angle et/ou de position de la lampe médicale, dans laquelle facultativement la détermination que la lampe médicale est en train d'être repositionnée comprend la détermination que le changement d'angle et/ou de position de la lampe médicale dépasse un ou plusieurs seuils prédéterminés.

15. Lampe médicale (12, 200, 302) selon l'une quelconque des revendications 8 à 14, dans laquelle la lampe médicale comprend un appareil de prise de vues (213), dans laquelle facultativement l'appareil de prise de vues est commandé par un dispositif de commande d'appareil de prise de vues externe (313), et le fait d'empêcher l'ajustement de l'au moins une fonction comprend le fait d'empêcher la communication de consignes au dispositif de commande d'appareil de prise de vues externe.

16. Produit programme d'ordinateur comprenant une ou plusieurs parties de code logiciel pour la commande d'au moins une caractéristique d'une lumière d'éclairage fournie par une lampe médicale (12, 200, 302), les une ou plusieurs parties de code logiciel comprenant des instructions qui, lors de leur exécution par un dispositif de commande (204, 307, 600) d'une lampe médicale, amènent le dispositif de commande à réaliser le procédé selon l'une quelconque des revendications 1 à 7.
